# EUROPEAN PATENT SPECIFICATION

(11) **EP 1 600 509 B1**
(45) Date of publication and mention of the grant of the patent: **02.11.2011**
(21) Application number: 04716353.0
(22) Date of filing: 02.03.2004
(51) Int. Cl.: C12N 15/82, A01H 5/00, A01H 5/10, C07H 3/06

(54) **TRANSGENIC PLANT HAVING FRUCTOOLIGOSACCHARIDE ACCUMULATED THEREIN AND PROCESS FOR CONSTRUCTING THE SAME**
TRANSGENE PFLANZE MIT DARIN ANGEREICHERTEM FRUCTOOLIGOSACCHARID UND VERFAHREN ZUR KONSTRUKTION DAVON
PLANTE TRANSGENIQUE AYANT UN FRUCTOOLIGOSACCHARRIDE ACCUMULE ET SON PROCEDE D'OBTENTION

(30) Priority: 03.03.2003 JP 2003055220
(43) Date of publication of application: 30.11.2005
(73) Proprietor: Meiji Seika Kaisha Ltd., Tokyo 104-8002 (JP)
(72) Inventor: NAKAMURA, Hirofumi, Meiji Seika Kaisha, Ltd., Sakado-shi, Saitama 3500289 (JP); KUBOTA, Hidetoshi, Meiji Seika Kaisha, Ltd., Sakado-shi, Saitama 3500289 (JP); KAWAI, Shinya, c/o Tokyo Uni. Agri. & Tech., Fuchu-shi, Tokyo 1838509 (JP); MITSUNARI, Takashi, c/o Tokyo Uni. Agri. & Tech., Fuchu-shi, Tokyo 1838509 (JP); FUKUTOMI, Daisuke, c/o Tokyo Uni. Agri. & Tech., Fuchu-shi, Tokyo 1838509 (JP)
(74) Representative: Minderop, Ralph H.
(86) International application number: PCT/JP2004/002564
(87) International publication number: WO 2004/078966

(56) References cited:
- WO-A1-96/01904
- WO-A1-97/34004
- WO-A2-01/36622
- WO-A2-03/000854
- US-A1- 2003 213 013
- HELLWEGE E M ET AL: "TRANSGENIC POTATO TUBERS ACCUMULATE HIGH LEVELS OF 1-KESTOSE AND NYSTOSE: FUNCTIONAL IDENTIFICATION OF A SUCROSE SUCROSE 1- FRUCTOSYLTRANSFERASE OF ARTICHOKE (CYNARA SCOLYMUS) BLOSSOM DISCS" PLANT JOURNAL, BLACKWELL SCIENTIFIC PUBLICATIONS, OXFORD, GB, vol. 12, no. 5, November 1997 (1997-11), pages 1057-1065, XP002071420 ISSN: 0960-7412
- REHM JOCHEN ET AL: "Production of 1-kestose in transgenic yeast expressing a fructosyltransferase from Aspergillus foetidus" JOURNAL OF BACTERIOLOGY, WASHINGTON, DC, US, vol. 180, no. 5, March 1998 (1998-03), pages 1305-1310, XP002164958 ISSN: 0021-9193
- "E.C. 3.2.1.26, beta-fructofuranosidase"[Online] XP002395469 KEGG ENZYME Retrieved from the Internet: URL:http://www.genome.jp/dbget-bin/www_bge t?ec:3.2.1.26> [retrieved on 2006-08-21]
- HELLWEGE, E.M: ET AL: 'Transgenic potato tubers accumulate high levels of 1-kestose and nystose: functional identification of sucrose sucrose 1-fructosyltransferase of artichoke (Cynara scolymus) blossom discs' PLANT J, vol. 12, no. 5, 1997, pages 1057 - 1065, XP002071420
- SEVENIER, R. ET AL: 'High level fructan accumulation in a transgenic sugar beet' NAT BIOTECHNOL, vol. 16, no. 9, 1998, pages 843 - 846, XP000877303
- VAN DER MEER, I.M. ET AL: 'Cloning of the fructan biosynthesis pathway of jerusalem artichoke' PLANT J. vol. 15, no. 4, 1998, pages 489 - 500, XP002096401

### SEQUENCE LISTING

<110> MEIJI SEIKA KAISHA. LTD.
<120> Transgenic plants modified to accumulate fructooligosaccharides and production thereof
<130> MEJ-708
<150> JP 2003-055220
   < 151 > 2003-03-03
<160> 6
<170> PatentIn version 3.1
<210> 1
   <211> 1965
   <212> DNA
   <213> Aspergillus niger ACE-2-1(ATCC20611)
<400> 1
   atgaagctca ccactaccac cctggcgctc gccaccggcg cagcagcagc agaagcctca      60
   taccacctgg acaccacggc cccgccgccg accaacctca gcaccctccc caacaacacc     120
   ctcttccacg tgtggcggcc gcgcgcgcac atcctgcccg ccgagggcca gatcggcgac     180
   ccctgcgcgc actacaccga cccatccacc ggcctcttcc acgtggggtt cctgcacgac     240
   ggggacggca tcgcgggcgc caccacggcc aacctggcca cctacaccga tacctccgat     300
   aacgggagct tcctgatcca gccgggcggg aagaacgacc ccgtcgccgt gttcgacggc     360
   gccgtcatcc ccgtcggcgt caacaacacc cccaccttac tctacacctc cgtctccttc     420
   ctgcccatcc actggtccat cccctacacc cgcggcagcg agacgcagtc gttggccgtc     480
   gcgcgcgacg gcggccgccg cttcgacaag ctcgaccagg gccccgtcat cgccgaccac     540
   cccttcgccg tcgacgtcac cgccttccgc gatccgtttg tcttccgcag tgccaagttg     600
   gatgtgctgc tgtcgttgga tgaggaggtg gcgcggaatg agacggccgt gcagcaggcc     660
   gtcgatggct ggaccgagaa gaacgccccc tggtatgtcg cggtctctgg cggggtgcac 720
   ggcgtcgggc ccgcgcagtt cctctaccgc cagaacggcg ggaacgcttc cgagttccag 780
   tactgggagt acctcgggga gtggtggcag gaggcgacca actccagctg gggcgacgag 840
   ggcacctggg ccgggcgctg ggggttcaac ttcgagacgg ggaatgtgct cttcctcacc 900
   gaggagggcc atgaccccca gacgggcgag gtgttcgtca ccctcggcac ggaggggtct 960
   ggcctgccaa tcgtgccgca ggtctccagt atccacgata tgctgtgggc ggcgggtgag 1020
   gtcggggtgg gcagtgagca ggagggtgcc aaggtcgagt tctccccctc catggccggg 1080
   tttctggact gggggttcag cgcctacgct gcggcgggca aggtgctgcc ggccagctcg 1140
   gcggtgtcga agaccagcgg cgtggaggtg gatcggtatg tctcgttcgt ctggttgacg 1200
   ggcgaccagt acgagcaggc ggacgggttc cccacggccc agcaggggtg gacggggtcg 1260
   ctgctgctgc cgcgcgagct gaaggtgcag acggtggaga acgtcgtcga caacgagctg 1320
   gtgcgcgagg agggcgtgtc gtgggtggtg ggggagtcgg acaaccagac ggccaggctg 1380
   cgcacgctgg ggatcacgat cgcccgggag accaaggcgg ccctgctggc caacggctcg 1440
   gtgaccgcgg aggaggaccg cacgctgcag acggcggccg tcgtgccgtt cgcgcaatcg 1500
   ccgagctcca agttcttcgt gctgacggcc cagctggagt tccccgcgag cgcgcgctcg 1560
   tccccgctcc agtccgggtt cgaaatcctg gcgtcggagc tggagcgcac ggccatctac 1620
   taccagttca gcaacgagtc gctggtcgtc gaccgcagcc agactagtgc ggcggcgccc 1680
   acgaaccccg ggctggatag ctttactgag tccggcaagt tgcggttgtt cgacgtgatc 1740
   gagaacggcc aggagcaggt cgagacgttg gatctcactg tcgtcgtgga taacgcggtt 1800
   gtcgaggtgt atgccaacgg gcgctttgcg ttgagcacct gggcgagatc gtggtacgac 1860
   aactccaccc agatccgctt cttccacaac ggcgagggcg aggtgcagtt caggaatgtc 1920
   tccgtgtcgg aggggctcta taacgcctgg ccggagagaa attga 1965
<210> 2
   <211> 27
   <212> DNA
   <213> Artificial Sequence
<220>
   <223> Description of Artificial Sequence: Primer CaMV35S
<400> 2
   ttcctctata taaggaagtt catttca 27
<210> 3
   <211> 27
   <212> DNA
   <213> Artificial Sequence
<220>
   <223> Description of Artificial Sequence: Primer nos-Ter.
<400> 3
   ataatttatc ctagtttgcg cgctata 27
<210> 4
   <211> 30
   <212> DNA
   <213> Artificial Sequence
<220>
   <223> Description of Artificial Sequence: Primer SPOA1S
<400> 4
   taagcttaat ttactaattt ggggttttac 30
<210> 5
   <211> 27
   <212> DNA
   <213> Artificial Sequence
<220>
   <223> Description of Artificial Sequence: Primer FFaseRev
<400> 5
   agagcccctc cgacacggag acattcc 27
<210> 6
   <211> 30
   <212> DNA
   <213> Artificial Sequence
<220>
   <223> Description of Artificial Sequence: Primer SPOB1S2
<400> 6
   taagctttag gttcactcac cttaagtttc 30

## Description

### TECHNICAL FIELD

The present invention relates to a transgenic plant which accumulates fructooligosaccharides with a high purity and a high content, and a process for producing the same. More particularly, the present invention relates to a process for producing a transgenic plant by expressing a gene encoding β-fructofuranosidase (such as β-fructofuranosidase derived from a mold) in a plant, to accumulate at least one of 1-kestose, nystose, or 1-fructofuranosylnystose.

### BACKGROUND ART

Fructooligosaccharides are oligosaccharides in which one or more fructoses are bonded to sucrose through a β2-1 bond, and the reducing terminus thereof is glucose. It is known that fructooligosaccharides have various physiological activities, for example, non-cariogenicity, an activity of promoting the growth of bifidobacteria, an activity of improving the metabolism of lipids such as cholesterol, or an activity of regulating immunity, and thus, fructooligosaccharides are industrially useful as functional food material. In nature, fructooligosaccharides are widely distributed in plants. It is known that fructooligosaccharides are contained in, for example, asparagus, onion, Jerusalem artichoke, or honey, but the content is low (for example, approximately 2.8 g/100 g onion)

The Plant Journal (1997), 1057-1065 describes an artichoke sucrose sucrose 1-fructosyltransferase capable of converting sucrose to kestose. It likewise shows transgenic potato tubers expressing Cy21 under the CaMV promotor which accumulate high levels of 1-ketose and nystose.

Recently, a mass production technique of obtaining fructooligasaccharides from sucrose by utilizing a transfer reaction catalyzed by β-fructofuranosidase derived from a microorganism was established, and used industrially. The industrially used β-fructofuranosidase derived from a mold is **characterized in that** it exhibits a high transfer activity and few isomers are produced. Therefore, fructooligosaccharides having a polymerization degree of 3 to 6 can be efficiently produced by performing an enzyme reaction using sucrose as a substrate. In addition, the mold β-fructofuranosidase was modified to obtain a β-fructofuranosidase variant capable of producing fructooligosaccharides having a different composition from that of fructooligosaccharides produced by the original β-fructofuranosidase [WO97/34004 (patent reference 1)].

With respect to techniques of transforming plants with a specific gene, various crops to which useful features were added have been generated, since a gene was introduced into tobacco using *Agrobacterium tumefaciens.* Further, attempts to accumulate useful substances in plants were carried out, and an accumulation of fructooligosaccharides in a plant is known [WO96/01904 (patent reference 2), WO03/00854 (patent reference 3), and "Nature Biotechnology", vol. 16, 1998, p. 843-846 (non-patent reference 1)].

However, an amount of fructooligosaccharides produced in the above references was very small, and isomers of fructooligosaccharides, for example, neokestose or 6-kestose as isomers of 1-kestose, were produced [patent reference 2, p. 56-59, Fig. 17A and Fig. 17B, and patent reference 3]. Further, the amounts of 1-kestose in the roots and leaves of a transformant from a beet, in which a storage sugar is sucrose, were 73.8 µmol/g FW (3.7%) and approximately 0.1 µmol/g FW, respectively (non-patent reference 1). (patent reference 1) International Publication No. 97/34004 (patent reference 2) International Publication No. 96/01904 (patent reference 3) International Publication No. 03/00854 (non-patent reference 1) "Nature Biotechnology", vol. 16, 1998, p. 843-846

### DISCLOSURE OF THE INVENTION

The conventional process for producing fructooligosaccharides by fermentation using β-fructofuranosidase derived from a microorganism has not only a problem of high production costs, but also a problem of reduction in physiological activities (such as prebiotic health effects, non-cariogenicity, or low calorie) of oligosaccharides, since a monosaccharide (glucose) is produced as a by-product. Therefore, a step of removing monosaccharides by a chromatographic fractionation or the like is required, and thus, the production costs are further increased. To solve such problems, a process for efficiently producing fructooligosaccharides with a high purity is desired.

Attempts to accumulate fructooligosaccharides in plants were carried out, but no transgenic plants which accumulate fructooligosaccharides, particularly 1-kestose, nystose, and/or 1-fructofuranosylnystose, with a high purity and a high content have been reported.

The present inventors used a gene encoding β-fructofuranosidase derived from *Aspergillus niger,* in which the expression thereof in plants had not been reported, and conducted intensive studies into the preparatoin of gene constructs capable of transforming plants. Further, the present inventors transformed plants with the gene constructs and regenerated plants, to produce transgenic plants capable of accumulating fructooligosaccharides. Although naturally-occurring tobaccos do not produce fructooligosaccharides, a transgenic plant of the present invention, a transgenic tobacco, has novel features in which approximately 3 to 4 µmol/g of 1-kestose is produced and accumulated, and isomers of 1-kestose (i.e., 6-kestose and neokestose) are not detected. Another transgenic plant of the present invention, a transgenic beet, has novel features in which approximately 0.15 µmol/g of 1-kestose is produced and accumulated in the leaves, and isomers of 1-kestose (i.e., 6-kestose and neokestose) are not detected.

Therefore, an object of the present invention is to provide a process for producing a transgenic plant which accumulates one or more fructooligosaccharides, comprising transforming a plant with a gene construct comprising a gene encoding β-fructofuranosidase capable of converting sucrose into one or more fructooligosaccharides, a transgenic plant produced by the process, and a progeny plant and a seed thereof. Further, another object of the present invention is to provide a process for manufacturing one or more fructooligosaccharides, using the transgenic plant of the present invention, or the progeny plant or the seed thereof.

The present invention includes the following inventions:
(1) a process for producing a transgenic plant which accumulates a fructooligosaccharide, comprising:
   transforming a plant with a gene construct comprising a gene encoding β-fructofuranosidase capable of converting sucrose into a fructooligosaccharide,
      wherein the gene encoding β-fructofuranosidase is derived from *Aspergillus* niger is selected from the group consisting of:
      (a) a gene consisting of the nucleotide sequence of SEQ ID NO: 1,
      (b) a gene comprising the nucleotide sequence of SEQ ID NO: 1,
(2) the process of (1) wherein the gene construct comprises a gene which encodes β-fructofuranosidase and is operably linked to a constitutive promoter, an organ-specific promoter, or a developmental-specific promoter,
(3) the process of (2), wherein the promoter is selected from the group consisting of:
   (i) a CaMV35S promoter,
   (ii) a sweet potato sporamin A promoter, and
   (iii) a sweet potato sporamin B promoter,
(4) the process of any one of (1) to (3), wherein the transgenic plant is a dicotyledonous plant or a monocotyledonous plant,
(5) the process of (4), wherein the transgenic plant is a plant belonging to Solanaceae, Chenopodiaceae, or Gramineae (Poaceae),
(6) the process of (5), wherein the transgenic plant is Nicotiana sp., Beta sp. or Saccharum sp.,
(7) a transgenic plant produced by the process of any one of (1) to (6), or a progeny plant thereof,
(8) a seed of the transgenic plant or progeny thereof of (7), and
(9) a process for manufacturing a fructooligosaccharide, comprising:
   cultivating the transgenic plant or progeny thereof of (7) or the seed of (8), and
   collecting a fructooligosaccharide accumulated in the plant body.

### BEST MODE FOR CARRYING OUT THE INVENTION

### Gene encoding β-fructofuranosidase

β-fructofuranosidase is a hydrolase which is classified into EC3.2.1.26 in the IUBMB (Nomenclature Committee of the International Union of Biochemistry and Molecular Biology) classification. β-fructofuranosidase which may be used in the present invention is not particularly limited, so long as it has an activity of converting sucrose into one or more fructooligosaccharides.

The term "fructooligosaccharide(s)" as used herein means a fructan having a polymerization degree of 3 or more in which one or more fructoses are bonded to sucrose through a β2→1 bond, and the reducing terminus thereof is glucose. Fructans having polymerization degrees of 3, 4, and 5 are 1- kestose, nystose, and 1-fructofuranosylnystose, respectively. An object of the present invention is to efficiently produce one or more fructooligosaccharides having polymerization degrees of particularly 3 to 5.

In the present invention, the selection of a gene encoding β-fructofuranosidase is important. β-fructofuranosidases derived from molds have preferred features in which fructooligosaccharides can be efficiently produced and few isomers are detected, since they exhibit a high specific activity and can act across a wide pH range and a wide temperature range, in comparison with fructooligosaccharide-generating enzymes derived from plants or bacteria.

As genes encoding preferred β-fructofuranosidases, there may be mentioned, for example, genes derived from a microorganism belonging to genus *Aspergillus,* genus *Penicillium,* or genus *Scopulariopsis.* Genes derived from *Aspergillus niger* are preferable, and include, for example, a gene (SEQ ID NO: 2 in WO97/34004) encoding an enzyme derived from *Aspergillus niger* ACE-2-1 (ATCC20611), a gene (SEQ ID NO: 12 in WO97/34004) encoding an enzyme derived from *Penicillium roqueforti* (IAM7254), and a gene (SEQ ID NO: 14 in WO97/34004) encoding an enzyme derived from *Scopulariopsis brevicaulis* (IFO4843) [the above three genes are disclosed in WO97/34004], and a gene (SEQ ID NO: 2 in WO99/13059) encoding an enzyme derived from *Penicillium roqueforti* (IAM7254), and a gene (SEQ ID NO: 4 in WO99/13059) encoding an enzyme derived from *Scopulariopsis brevicaulis* (IFO4843) [the above two genes are disclosed in WO99/13059].

Further, the gene encoding β-fructofuranosidase may be selected from:
(a) a gene consisting of the nucleotide sequence of SEQ ID NO: 1,
(b) a gene comprising the nucleotide sequence of SEQ ID NO: 1,
(c) a gene comprising a nucleotide sequence in which one or plural nucleotides are deleted, substituted, or added in the nucleotide sequence of SEQ ID NO: 1, and encoding β-fructofuranosidase capable of converting sucrose into one or more fructooligosaccharides, or
(d) a gene comprising a nucleotide sequence having an 85% or more homology with that of SEQ ID NO: 1, and encoding β-fructofuranosidase capable of converting sucrose into one or more fructooligosaccharides.

The above gene (b) is not particularly limited, so long as it comprises the nucleotide sequence of SEQ ID NO: 1 and encodes β-fructofuranosidase capable of converting sucrose into one or more fructooligosaccharides. As the gene (b), there may be mentioned, for example, a gene consisting of a nucleotide sequence in which a nucleotide sequence encoding an appropriate marker sequence and/or a partner for fusion is added to the 5' terminus and/or the 3' terminus of the nucleotide sequence of SEQ ID NO: 1.

As the marker sequence, for example, a sequence for easily carrying out a confirmation of polypeptide expression, a confirmation of intracellular localization thereof, or a purification thereof may be used. As the sequence, there may be mentioned, for example, a FLAG tag, a hexa-histidine tag, a hemagglutinin tag, or a myc epitope.

As the partner for fusion, there may be mentioned, for example, a polypeptide for purification [for example, glutathione S-transferase (GST) or a fragment thereof], a polypeptide for detection [for example, hemagglutinin or β-galactosidase α peptide (LacZ α), or a fragment thereof], or a polypeptide for expression (for example, a signal sequence).

In the gene (c), the number of nucleotides to be deleted, substituted, or added is, for example, 1 to 60, preferably 1 to 30, more preferably 1 to 15.

More particularly, genes encoding variants obtainable by procedures described in Examples D1 to D11 of WO97/34004 may be used. The variants of β-fructofuranosidase derived from *Aspergillus niger* ATCC20611 include variants F170W, G300W, H313K, E386K, F170W+G300W, F170W+G300W+H313R, G300W+H313K, G300V+H313K, G300E+H313K, G300D+H313K, F170W+G300W+H313K, and F170W+G300V+H313K. In this connection, each name of the variants indicates the original amino acid, the nucleotide number, and an amino acid replaced by substitution. For example, the term "F170W" means that the original amino acid, phenylalanine (F) at the 170th position was replaced with tryptophan (W). Further, multiple mutations are represented by the symbol "+". For example, the term "F170W+G300V+H313K" means that phenylalanine, glycine (G), and histidine (H) at the 170th, 300th, and 313th positions were replaced with tryptophan, valine (V), and lysine (K), respectively.

The gene encoding β-fructofuranosidase can be prepared, preferably, in accordance with procedures described in WO97/34004 or WO99/13059. More particularly, by reference to Example A and Example D1 of WO97/34004, plasmid pAN120 (Fig. 6 in WO97/34004) can be prepared and digested with *Bam*HI to obtain a cDNA of β-fructofuranosidase (FFase) derived from *Aspergillus niger* ATCC20611.

In the gene (d), the homology with the nucleotide sequence of SEQ ID NO: 1 is preferably 90% or more, more preferably 95% or more, still further preferably 98% or more, most preferably 99% or more. The term "homology" as used herein means a value obtained by a known program for a homology search, BLAST (Basic local alignment search tool; Altschul,S.F. et al., J.Mol.Biol., 215, 403-410, 1990).

### Gene construct

The gene encoding β-fructofuranosidase, obtainable by the above-mentioned procedures, or a plasmid containing the gene may be used to prepare a gene construct (such as a binary vector or plasmid for plants) capable of being expressed in plants.

In addition to the gene encoding β-fructofuranosidase and an appropriate promoter active in a plant body, the gene construct which may be used in the present invention may contain, for example, an appropriate terminator (such as a nopaline synthase gene terminator or a CaMV35S terminator), an element useful for an expression re gulation, and/or an appropriate marker gene for a transformant selection (for example, drug-resistant genes such as a kanamycin-resistant gene, a hygromycin-resistant gene, or a G418 resistant gene).

As the appropriate promoter active in a plant body, there may be mentioned, for example, constitutive promoter, an organ-specific promoter, or a devel opmental-specific promoter. The constitutive promoter is a promoter in which a constant expression is performed regardless of the organs or growth conditions of a plant. As the constitutive promoter, for example, a cauliflower mosaic virus 35S promoter may be used. The organ-specific promoter is a promoter in which an expression is specifically performed in a specific organ (such as roots, leaves, or stems). As the organ-specific promoter, a sporamin A promoter or a sporamin B promoter may be used. The developmental-specific promoter is a promoter in which an expression is specifically performed in a specific developmental stage (such as a germination period or a bearing period). The promoter may be appropriately selected in accordance with, for example, a host to be used, or an organ, tissue, and/or developmental stage to be expressed. When the promoter used in the present invention is introduced into a plant, an RNA polymerase specifically binds to the promoter, and a transcription begins in the downstream direction. Preferred promoters include:
(i) a cauliflower mosaic virus 35S promoter (a CaMV35S promoter),
(ii) a sweet potato sporamin A promoter, and
(iii) a sweet potato sporamin B promoter.

For example, when the gene encoding β-fructofuranosidase is expressed in tuberous roots, sweet potato sporamin promoters are preferable. As a storage protein, sweet potato (*Ipomoea batatas*) produce sporamins, which account for 60% to 80% of the total soluble proteins contained in sweet potato tuberous roots. Genes encoding the sporamins form a multigene family, and are classified into sporamin A genes and sporamin B genes on the basis of homologies. Each gene has a promoter which is specifically expressed in tuberous roots. The sporamin promoters are induced by saccharides, particularly sucrose.

The procedure for constructing the gene construct which may be used in the present invention is not particularly limited, but it may be prepared, for example, by the following procedure. A cDNA of β-fructofuranosidase derived from *Aspergillus niger* is inserted into the *Bam*HI site located downstream of the CaMV35S promoter in plasmid pBI121 (Clonetech) to obtain a binary vector. If necessary, a β-glucuronidase gene may be removed. In the binary vector, the CaMV35S promoter is linked to the upstream side of the β-fructofuranosidase gene, and a heparin synthase gene terminator derived from a Ti plasmid is linked to the downstream side of the β-fructofuranosidase gene. Therefore, the β-fructofuranosidase gene on the binary vector can be expressed in a plant. Further, the binary vector contains a kanamycin-resistant gene, and can give a kanamycin resistance to a plant or a microorganism such as Escherichia coli.

### Introduction and expression of β-fructofuranosidase gene in plant

A method which may be used for introducing a gene into a plant is not particularly limited, and any method known to those skilled in the art as a gene introduction method for plant cells or a plant can be used. For example, as a preferred embodiment of the present invention, an agrobacterium may be used for introducing the gene construct into a plant. When an agrobacterium is used for transforming a plant, border sequences of a T-DNA region can be linked at the sites adjacent to the nucleotide sequence to be introduced. An appropriate procedure for constructing a vector for transformation using such a T-DNA is known to those skilled in the art.

As other embodiments, there may be mentioned, for example, an introduction method using calcium and/or polyethylene glycol, an electroporation method, or a particle gun method.

The plant in which the gene encoding β-fructofuranosidase may be introduced is not particularly limited, but a dicotyledonous plant or a monocotyledonous plant is preferable. A plant belonging to Solanaceae or Chenopodiaceae and a plant belonging to Gramineae (Poaceae) are more preferable as the dicotyledonous plant and the monocotyledonous plant, respectively. A plant belonging to Nicotiana sp., Beta sp. or Saccharum sp. is still further preferable. Tobacco (*Nicotiana tabacum*)*,* beet (sugar beet: *Beta vulgaris* var. *rapa,* table beet: *Beta vulgaris* var. *rubra,* chard: *Beta vulgaris* var. *vulgaris,* or Beta vulgaris alba: *Beta vulgaris* var. *alba*)*,* or sugar cane (*Saccharum officinarum*)*,* or protoplasts thereof are most preferable. Beet and sugar cane are sucrose-storage plants, and advantageous effects may be obtained in the production of fructooligosaccharides, by expressing the β-fructofuranosidase gene in an organ or tissue for sucrose storage thereof.

As a method for introducing the gene construct containing the gene encoding β-fructofuranosidase according to the present invention into a chromosome of a plant, for example, a leaf disk method (Horsh et al., Science, 227, 1229-1232, 1985) is preferable. More particularly, *Agrobacterium tumefaciens* is cultivated with shaking in a YEP liquid medium supplemented with streptomycin, for example, at 28°C for 8 to 9 hours, and protoplasts (competent cells) are prepared by a conventional method. The gene construct containing the gene encoding β-fructofuranosidase is added to the competent cells, and the whole is mixed gently and allowed to stand on ice. The mixture is transferred to a cuvette with electrodes at a 2 mm width (Gene Pluser/*E. Coli* Pulses^{™} Cuvette, BIO-RAD), and electroporation is carried out by an electroporation device [for example, GENE PULSER(R)II system, BIO-RAD] in accordance with a manual attached thereto. The treated mixture, together with the YEP liquid medium, are cultivated at 28 °C for 2 to 4 hours under stationary conditions, and further cultivated in an LB medium supplemented with an antibiotic such as kanamycin, to obtain transformants.

The transformants are cultivated in the YEP liquid medium, and the culture liquid is added to leaf disks obtained from leaves of a plant cultivated under sterile conditions. Cultivation is carried out in a differentiation medium to form and grow calli. As the differentiation medium for plants, known media such as an MS medium (Murashinge and Skoog, Physiol. Plant., 15, 473-497, 1962) may be used. A differentiation medium for selection may be used to select desired calluses. For example, a medium supplemented with 50 mg/L to 200 mg/L of kanamycin may be used.

Further, a root differentiation medium prepared by adding kanamycin or the like to a known medium such as the MS medium may be used to regenerate a plant body. A shoot may be transferred and cultivated to obtain a plant body (transgenic plant). Seeds of the plant may be cultivated to obtain progeny plants and seeds.

The term "plant" as used herein, for example, transgenic plant or progeny plant, includes not only a plant body as the whole of a living body, but also a part thereof, such as organs (for example, a leaf, stem, root, flower, or fruit), tissues, and cells.

### Collection and analysis of fructooligosaccharides contained in transgenic plant

The collection and analysis of fructooligosaccharides contained in the transgenic plant of the present invention may be carried out as described below. Each organ (such as roots, stems, or leaves) of a plant is ground in liquid nitrogen, and a desired amount of powder is weighed. A determined amount of distilled water is added to the powder, and sufficiently stirred. The supernatant is collected by centrifugation, and analyzed by known methods such as thin layer chromatography or high performance liquid chromatography to confirm a generation and accumulation of fructooligosaccharides contained in the plant.

### EXAMPLES

The present invention now will be further illustrated by, but is by no means limited to, the following Examples.

### Example 1: Introduction of β-fructofuranosidase gene into tobacco

### (1) Preparation of binary vector containing CaMV35S promoter

Plasmid pAN120 (Fig. 6 in WO97/34004) prepared by reference to Example A and Example D1 of WO97/34004 was digested with *Bam*HI to obtain a *Bam*HI-*Bam*HI fragment of approximately 1.9 kb containing a cDNA (SEQ ID NO: 1) of β-fructofuranosidase (FFase) derived from *Aspergillus niger* ATCC20611. Plasmid pBI121 (Clonetech), which had been digested with *Bam*HI, was ligated to the *Bam*HI-*Bam*HI fragment containing the FFase cDNA by a DNA Ligation Kit Ver.2 (Takara Shuzo), to obtain a binary vector in which the FFase gene was inserted into the downstream side of a CaMV35S promoter in pBI121.

### (2) Preparation of binary vector containing sweet potato sporamin A promoter

A sweet potato sporamin A promoter was prepared in accordance with the method described in Hattori, T., and Nakamura, K, "Plant Mol. Biol.", Vol. 11, 1988, p.417-426. The sporamin A promoter was digested with HindIII to obtain a HindIII-HindIII fragment (approximately 1 kbp) of the sporamin A promoter.

Plasmid pBI101 (Clonetech) was digested with SmaI and SacI, and was blunted and self-ligated to obtain plasmid pBI101-GUS from which a β-glucuronidase gene was removed.

The plasmid pBI101-GUS, which had been digested with *Hind*III, was ligated to the *Hind*III-*Hind*III fragment of the sporamin A promoter by the DNA Ligation Kit Ver.2. The ligated plasmid was digested with *Bam*HI, and ligated to the *Bam*HI-*Bam*HI fragment containing the FFase cDNA, prepared in Example 1(1), by the DNA Ligation Kit Ver.2, to prepare a binary vector in which the sporamin A promoter was inserted into the HindIII site of pBI101-GUS and the FFase gene was inserted into the *Bam*HI site located downstream thereof.

### (3) Preparation of vector containing sweet potato sporamin B promoter

A sweet potato sporamin B promoter was prepared in accordance with the method described in Hattori, T., and Nakamura, K, "Plant Mol. Biol.", Vol. 11, 1988, p.417-426. The sporamin B promoter was digested with HindIII and PstI to obtain a HindIII-PstI fragment (approximately 0.75 kbp) of the sporamin B promoter. The fragment was inserted into the multi-cloning site of plasmid pBlueScript KS(-). The resulting plasmid was digested with *Bam*HI, and ligated to the *Bam*HI-*Bam*HI fragment containing the FFase cDNA, prepared in Example 1(1), by the DNA Ligation Kit Ver.2. The resulting plasmid was digested with HindIII and XbaI to obtain a fragment of approximately 2.7 kbp containing the sporamin B promoter and the FFase gene located downstream thereof. The plasmid pBI101-GUS, prepared in Example 1(2), was digested with HindIII and XbaI, and ligated to a fragment of approximately 2.7 kbp by the DNA Ligation Kit Ver.2, to obtain a binary vector in which the sporamin B gene and the FFase gene located downstream thereof were inserted into the *Hin*dIII-*Xba*I site of pBI101-GUS.

### (4) Gene introduction into tobacco and regeneration of plant

*Agrobacterium tumefaciens* LBA4404 (Plasmid, Vol. 7, 1982, p.15) was cultivated with shaking in 250 mL of a YEP liquid medium supplemented with streptomycin at 28°C for 8 to 9 hours (until O.D.600 = 0.5).

### Composition of YEP liquid medium (g/100 mL)

| | |
|---|---|
| Bacto-peptone | 1 g |
| Bacto-yeast extracts | 1 g |
| Sodium chloride | 0.5 g |

Cells were collected by centrifugation, and suspended in 200 mL of an ice-cold 10% glycerol solution [9.31%(W/V) sucrose, 10%(V/V) glycerol, and 1 mmol/L magnesium chloride]. The procedure was repeated three times. Further, 20 to 30 mL of the ice-cold 10% glycerol solution was added, and the cells were suspended and centrifuged. The cells were suspended in 400 to 600 µL of the ice-cold 10% glycerol solution, and the suspension was quick-frozen with liquid nitrogen to prepare competent cells.

Each (1 µL) of three binary vector DNAs prepared in Examples 1(1), 1(2), and 1(3) was added to the competent cells (50 µL), and mixed gently and allowed to stand in ice for 30 seconds or more. Each mixture was transferred to a cuvette with electrodes at a 2 mm width (Gene Pluser/*E. Coli* Pulser^{™} Cuvette, BIO-RAD), and electroporation was carried out by an electroporation device [GENE PULSER(R)II system, BIO-RAD] in accordance with a manual attached thereto. To each treated mixture, 1 mL of the YEP liquid medium was added, and a stationary cultivation was carried out at 28°C for 2 to 4 hours. A further cultivation in an LB medium supplemented with kanamycin was carried out, to obtain transformants transformed with each of three binary vector DNAs.

### Composition of LB medium (g/100 mL)

| | |
|---|---|
| Bacto-tryptone | 1 g |
| Bacto-yeast extracts | 0.5 g |
| Sodium chloride | 0.5 g |
| (kanamycin | 15 µg/mL) |

The obtained transformants were cultivated in the YEP liquid medium at 28°C with shaking to obtain each culture solution (cultured until O.D.550 = 1.0). Leaf disks (5 to 7 mm) were cut from sterile leaves of tobacco (*Nicotiana tabacum* Samsun NN strain) cultivated under sterile conditions, and dipped in an MS liquid medium. Each of the obtained culture solutions was added on the leaf disks and allowed to stand for 30 minutes.

### MS medium (mg/L) (pH 5.7)

| | |
|---|---|
| NH₄NO₃ | 1,650 |
| KNO₃ | 1,900 |
| CaCl₂·2H₂O | 440 |
| MgSO₄·7H₂O | 370 |
| KH₂PO₄ | 170 |
| H₃BO₃ | 6.2 |
| MnSO₄·4H₂O | 22.32 |
| ZnSO₄·7H₂O | 8.6 |
| KI | 0.83 |
| Na₂MoO₄·2H₂O | 0.25 |
| CuSO₄·5H₂O | 0.025 |
| CoCl₂·6H₂O | 0.025 |
| EDTA-Na₂ | 37.3 |
| FeSO_{4·}7H₂O | 27.8 |
| myoinositol | 100 |
| glycine | 2.0 |
| pridoxine hydrochloride | 0.5 |
| nicotinic acid | 0.5 |
| thiamine hydrochloride | 0.1 |
| sucrose | 30,000 |
| agar | 9 |

[Agar was not added when preparing the liquid medium.]

The surplus culture solutions were removed from the leaf disks using sterile filter papers, and the leaf disks were placed with the back thereof facing upward in an MS shoot differentiation medium A, and cultivated in the dark at 25°C for 3 to 4 days. The leaf disks were transferred to an MS shoot differentiation medium B, and cultivated at 25°C (16 hours in the light/8 hours in the dark). After 3 to 4 weeks, the leaf disks were transferred to another fresh MS shoot differentiation medium B. When shoots were grown to a length of approximately 1 cm, the shoots only were transferred to an MS shoot differentiation medium C, to obtain whole plant bodies. The obtained plant bodies were subcultured in MS agar media. In this connection, the MS shoot differentiation media A to C were prepared by adding the following compounds to the MS medium, respectively.

### MS shoot differentiation medium A

| | |
|---|---|
| streptomycin | 15 mg/L |
| kanamycin | 150 mg/L |

### MS shoot differentiation medium B

| | |
|---|---|
| carbenicillin | 500 mg/L |
| kanamycin | 150 mg/L |

### MS shoot differentiation medium C

| | |
|---|---|
| kanamycin | 100 mg/L |

### (5) Confirmation of β-fructofuranosidase gene introduction into tobacco plant

### (5a) Isolation of total DNAs from tobacco plant

Leaf tissues (50 to 100 mg) were prepared from tobacco plants obtained by using binary vectors prepared in Examples 1(1), 1(2), and 1(3), frozen at -80°C, and thawed by adding 100 µL of an extraction buffer.

### Extraction buffer

| | | |
|---|---|---|
| urea | | 5 mol/L |
| 2-mercaptoethanol | | 10 mmol/L |
| phenol | | 5%(v/v) |
| sterile water | | 1 volume |
| 2xstock solution for extraction buffer [composition] | | 1 volume |
| | NaCl | 0.6 mol/L |
| | Tris-HCl(pH7.5) | 0.1 mol/L |
| | EDTA(pH8.0) | 40 mmol/L |
| | SDS | 1%(w/v) |

The leaf tissues were ground by a sonicator for 1 or 2 minutes, and a DNeasy Plant Mini Kit (QIAGEN) was used in accordance with a manual attached thereto, to obtain tobacco total DNAs.

### (5b) Detection of introduced gene by PCR method

Primers were designed on the basis of sequences upstream and downstream of the region in which the β-fructofuranosidase gene was introduced.

As primers for detecting the plant obtained by using the binary vector prepared in Example 1(1), the following primers were designed on the basis of sequences of the CaMV35S promoter region (3' side) and the *nos*-Ter. region (5' side):
CaMV35S:
   5'-TTCCTCTATATAAGGAAGTTCATTTCA-3' (SEQ ID NO: 2)
nos-Ter:
   5'-ATAATTTATCCTAGTTTGCGCGCTATA-3' (SEQ ID NO: 3)

With respect to primers for detecting the plant obtained by using the binary vector prepared in Example 1(2), the SPOA1S primer used when preparing the sweet potato sporamin A promoter, and an FFaseRev primer designed on the basis of the downstream region of the β-fructofuranosidase gene sequence were used, as the upstream primer and the downstream primer, respectively.
SPOAIS:
   5'-TAAGCTTAATTTACTAATTTGGGGTTTTAC-3' (SEQ ID NO: 4)
FFaseRev:
   5'-AGAGCCCCTCCGACACGGAGACATTCC-3' (SEQ ID NO: 5)

With respect to primers for detecting the plant obtained by using the binary vector prepared in Example 1(3), the SPOB1S2 primer used when preparing the sweet potato sporamin B promoter, and the above FFaseRev primer (SEQ ID NO: 5) designed on the basis of the downstream region of the β-fructofuranosidase gene sequence were used, as the upstream primer and the downstream primer, respectively.
SPOBlS2:
   5'-TAAGCTTTAGGTTCACTCACCTTAAGTTTC-3' (SEQ ID NO: 6)

Each of the total DNAs prepared in Example 1(5a) was used as a template to carry out PCR, and amplified gene fragments were observed. From the total DNAs prepared from the plant obtained by using the binary vector prepared in Example 1(1), a fragment of approximately 4 kbp containing the β-fructofuranosidase gene (approximately 1.9 kbp) and the GUS gene (approximately 2 kbp) was confirmed. From the total DNAs prepared from the plant obtained by using the binary vector prepared in Example 1(2) or Example 1(3), a fragment of approximately 3 kbp containing the sporamin promoter (approximately 1 kbp) and the β-fructofuranosidase gene (approximately 1.9 kbp) was confirmed.

### Example 2: Analysis of fructooligosaccharides in tobacco plant

Fructooligosaccharides contained in the stem of the tobacco transformant obtained in accordance with the procedures described in Example 1(4) using the binary vector (containing the sweet potato sporamin B promoter) prepared in Example 1(3) were analyzed as follows. Similarly, the original tobacco strain (i.e., not transformed) was analyzed.

The lower portions (1.5 g fresh weight) of stems of tobacco plants grown to a height of 30 cm or more were ground in the presence of liquid nitrogen, and pure water (1.5 mL) was added thereto and stirred. After an incubation at 80°C to 85°C for 1.5 hours, the mixture was centrifuged, and the resulting supernatant was used for the following analysis. In the analysis by high performance liquid chromatography, a detector (Differential refractometer 410; Waters) and a column [Hibar Lichrocart 250-4 LiChrospher 100NH₂ (4 mm I.D. x 250 mm); Kanto Chemical Co., Inc.] were used. The analysis conditions were as follows:
mobile phase: acetonitrile:water = 72:28
flow rate: 1.0 mL/min.
temperature: 40°C
The results are shown in Table 1.

**Table 1**

| | tobacco (original strain) (µmol/gFW) | tobacco (transgenic plant of the present invention) (µmol/gFW) |
|---|---|---|
| fructose | 1.8 | 3.0 |
| glucose | 4.8 | 8.9 |
| sucrose | 15.7 | 9.8 |
| 1-kestose | 0.0 | 3.6 |
| nystose | 0.0 | 0.9 |

Although tobacco does not produce fructooligosaccharides, it was confirmed that 1-kestose (GF₂) and nystose (GF₃) as fructooligosaccharide components were remarkably produced and accumulated in the stems of tobacco transformed by the method of the present invention (i.e., transgenic plant). Further, isomers of 1-kestose, i.e., 6-kestose and neokestose, were not detected (not shown in Table 1).

### Example 3: Introduction of β-fructofuranosidase gene into beet and analysis of fructooligosaccharides in beet plant

The fructofuranosidase gene was introduced into a beet plant in accordance with the agrobacterium method as described in Example 1(4). As the β-fructofuranosidase gene to be introduced, the binary vector prepared in Example 1(1) was used.

The procedures from the gene introduction to the plant regeneration were carried out in accordance with the procedures described in Example 1(4), except for minor changes of media as described below. After the gene was introduced into leaf disks cut from beet leaves, the leaf disks were cultivated in an MS shoot differentiation medium D, and transferred to an MS shoot differentiation medium E. When shoots were grown to a length of approximately 1 cm, the shoots were transferred to the MS shoot differentiation medium C, to generate plant bodies. In this connection, the MS shoot differentiation media D and E were prepared by adding the following compounds to the MS medium, respectively.

### MS shoot differentiation medium D

| | |
|---|---|
| naphthalene acetic acid | 1 mg/L |
| streptomycin | 15 mg/L |
| kanamycin | 150 mg/L |

### MS shoot differentiation medium E

| | |
|---|---|
| benzyladenine | 1 mg/L |
| naphthalene acetic acid | 1 mg/L |
| streptomycin | 15 mg/L |
| kanamycin | 150 mg/L |

Leaves [0.3 gFW (fresh weight)] of beet plants were ground in the presence of liquid nitrogen, and pure water (1 mL) was added thereto and stirred. After an incubation at 80°C to 85°C for 1.5 hours, the mixture was centrifuged, and the resulting supernatant was used for the HPLC analysis, as described in Example 2. As a result, 0.15 µmol/gFW of 1-kestose was detected. Further, isomers of 1-kestose, i.e., 6-kestose and neokestose, were not detected.

### INDUSTRIAL APPLICABILITY

According to the present invention, transgenic plants which accumulate fructooligosaccharides with a high purity and a high content in the bodies thereof can be obtained, and fructooligosaccharides can be efficiently produced.

### FREE TEXT IN SEQUENCE LISTING

Features of "Artificial Sequence" are described in the numeric identifier <223> in the Sequence Listing. More particularly, the nucleotide sequences of SEQ ID NOS: 2 to 6 are primer CaMV35S, primer nos-Ter, primer SPOA1S, primer FFaseRev, and primer SPOB1S2, respectively.

## Claims

1. A process for producing a transgenic plant which accumulates a fructooligosaccharide, comprising:
transforming a plant with a gene construct comprising a gene encoding β-fructofuranosidase capable of converting sucrose into a fructooligosaccharide, wherein the gene encoding fructofuranosidase is derived from *Aspergillus niger,* and is selected from the group consisting of:
(a) a gene consisting of the nucleotide sequence of SEQ ID NO: 1, and
(b) a gene comprising the nucleotide sequence of SEQ ID NO:1.

2. The process according to claim 1, wherein the gene construct comprises a gene which encodes β-fructofuranosidase and is operably linked to a constitutive promoter, an organ-specific promoter, or a developmental-specific promoter.

3. The process according to claim 2, wherein the promoter is selected from the group consisting of:
(i) a CaMV35S promoter,
(ii) a sweet potato sporamin A promoter, and
(iii) a sweet potato sporamin B promoter.

4. The process according to any one of claims 1 to 3, wherein the transgenic plant is a dicotyledonous plant or a monocotyledonous plant.

5. The process according to claim 4, wherein the transgenic plant is a plant belonging to Solanaceae, Chenopodiaceae, or Gramineae (Poaceae).

6. The process according to claim 5, wherein the transgenic plant is Nicotiana sp., Beta sp. or Saccharum sp.

7. A transgenic plant produced by the process according to any one of claims 1 to 6, or a progeny plant thereof, wherein the progeny plant comprises the gene construct described in claim 1.

8. A seed of the transgenic plant or progeny thereof according to claim 7.

9. A process for manufacturing a fructooligosaccharide, comprising:
cultivating the transgenic plant or progeny thereof according to claim 7 or the seed according to claim 8, and
collecting a fructooligosaccharide accumulated in the plant body.

## Patentansprüche

1. Verfahren zur Erzeugung einer transgenen Pflanze, die Fructooligosaccharide anreichert, umfassend das Transformieren einer Pflanze mit einem Genkonstrukt, das ein β-Fructofuranosidase kodierendes Gen enthält, welches geeignet ist, Saccharose in ein Fructooligosaccharid umzuwandeln, wobei das β-Fructofuranosidase kodierende Gen von *Aspergillus niger* stammt und ausgewählt ist aus der Gruppe, bestehend aus
(a) einem Gen, bestehend aus der Nukleotidsequenz SEQ ID NO:1, und
(b) einem Gen, umfassend die Nukleotidsequenz SEQ ID NO:1.

2. Verfahren nach Anspruch 1, worin das Genkonstrukt ein Gen, das β-Fructofuranosidase kodiert, umfasst und funktionell mit einem konstitutiven Promotor, einem organspezifischen Promotor oder einem entwicklungsspezifischen Promotor verbunden ist.

3. Verfahren nach Anspruch 2, worin der Promotor ausgewählt ist aus der Gruppe, bestehend aus
(i) einem CaMV35S Promotor,
(ii) einem Sporamin A Promotor der Süßkartoffel und
(iii) einem Sporamin B Promotor der Süßkartoffel.

4. Verfahren nach einem der Ansprüche 1 bis 3, worin die transgene Pflanze eine zweikeimblättrige Pflanze oder eine einkeimblättrige Pflanze ist.

5. Verfahren nach Anspruch 4, worin die transgene Pflanze zu den Nachtschattengewächsen (Solanaceae), den Gänsefußgewächsen (Chenopodiaceae) oder den Süßgräsern (Gramineae oder Poaceae) gehört.

6. Verfahren nach Anspruch 5, worin die transgene Pflanze Nicotiana sp., Beta sp. oder Saccharum sp. ist.

7. Transgene Pflanze, erzeugt durch das Verfahren nach einem der Ansprüche 1 bis 6, oder eine Nachkommenpflanze davon, wobei die Nachkommenpflanze das in Anspruch 1 beschriebene Genkonstrukt umfasst.

8. Samen der transgenen Pflanze oder deren Nachkommen nach Anspruch 7.

9. Verfahren zur Herstellung eines Fructooligosaccharids, umfassend das Züchten der transgenen Pflanze oder deren Nachkommen nach Anspruch 7 oder des Samens nach Anspruch 8 und das Ernten des im Pflanzenkörper angereicherten Fructooligosaccharids.

## Revendications

1. Procédé de production d'une plante transgénique accumulant un fructooligosaccharide, comprenant :
la transformation d'une plante avec une construction génique comprenant un gène codant pour la β-fructofuranosidase capable de convertir le saccharose en un fructooligosaccharide, dans laquelle le gène codant pour la β-fructofuranosidase est issu de *Aspergillus niger,* et est choisi dans le groupe consistant en :
(a) un gène consistant en la séquence nucléotidique de SEQ ID NO : 1, et
(b) un gène comprenant la séquence nucléotidique de SEQ ID NO : 1.

2. Procédé selon la revendication 1, dans lequel la construction génique comprend un gène codant pour la β-fructofuranosidase et lié de manière fonctionnelle à un promoteur constitutif, un promoteur spécifique d'un organe, ou un promoteur spécifique du développement.

3. Procédé selon la revendication 2, dans lequel le promoteur est choisi parmi le groupe consistant en :
(i) un promoteur CaMV35S,
(ii) un promoteur sporamine A de la patate douce, et
(iii) un promoteur sporamine B de la patate douce.

4. Procédé selon l'une quelconque des revendications 1 à 3, dans lequel la plante transgénique est une plante dicotylédone ou une plante monocotylédone.

5. Procédé selon la revendication 4, dans lequel la plante transgénique est une plante appartenant aux Solanacées, Chénopodiacées, ou aux Graminées (Poacées).

6. Procédé selon la revendication 5, dans lequel la plante transgénique est Nicotiana sp., Beta sp. or Saccharum sp.

7. Plante transgénique produite par le procédé selon l'une quelconque des revendications 1 à 6, ou plante descendante de ladite plante transgénique, dans laquelle la plante descendante comprend la construction génique décrite dans la revendication 1.

8. Graine issue de ladite plante transgénique ou de ladite plante descendante selon la revendication 7.

9. Procédé de fabrication d'un fructo-oligosaccharide, comprenant :
la culture de ladite plante transgénique ou de ladite plante descendante selon la revendication 7 ou de la graine selon la revendication 8, et la récupération d'un fructo-oligosaccharide accumulé dans le corps de la plante.
